# FASCICULE DE BREVET EUROPEEN

(11) **EP 0 618 193 B1**
(45) Date de publication et mention de la délivrance du brevet: **21.10.1998**
(21) Numéro de dépôt: 94400579.2
(22) Date de dépôt: 17.03.1994
(51) Int. Cl.: C07D 209/42, A61K 31/395, C07D 453/06, C07D 409/08

(54) **Dérivés bicycliques azotés, en tant qu'inhibiteurs de prolyl-endopeptidase**
Bicyclische Stichstoff enthaltende Derivate als Prolyl-Endopeptidase Inhibitoren
Nitrogen containing bicyclic derivatives as prolyl-endopeptidase inhibitors

(30) Priorité: 24.03.1993 FR 9303363
(43) Date de publication de la demande: 05.10.1994
(73) Titulaire: ADIR ET COMPAGNIE, 92415 Courbevoie Cédex (FR)
(72) Inventeur: de Nanteuil, Guillaume, F-92150 Suresnes (FR); Portevin, Bernard, F-78990 Elancourt (FR); Remond, Georges, F-78000 Versailles (FR); Lepagnol, Jean, F-28210 Chaudon (FR); Heidet, Véronique, F-75012 Paris (FR)

(56) Documents cités:
- EP-A- 0 268 190
- EP-A- 0 320 753
- EP-A- 0 321 956
- FR-A- 2 681 864

## Description

La présente invention concerne de nouveaux dérivés bicycliques azotés, leur procédé de préparation et les compositions pharmaceutiques qui les contiennent.

Le vieillissement de la population par allongement de la durée de vie rend de plus en plus important le problème du vieillissement cérébral et des démences séniles liées à l'âge. C'est pourquoi, la recherche de nouveaux composés thérapeutiques pouvant s'opposer aux déficits de la mémoire et aux troubles neuropsychocomportementaux de la sénescence est devenue prioritaire.

A côté des neurotransmetteurs classiques (acétylcholine, norepinephrine) impliqués dans les fonctions de mémoire, il y a des neuropeptides tels que la vasopressine (AVP) ou la thyrotropin releasing hormone (TRH) qui exercent également des effets facilitateurs mnésiques en jouant un rôle de neuromodulation en plus de leur rôle périphérique ou endocrinien (Science, 211, 601, 1981).

Récemment, des études ont montré que les taux cérébraux de ces peptides diminuent significativement chez les patients atteints de démence sénile (Neurobiology, 36, 1133, 1986).

Encore plus récemment, il a été observé que l'activité de la prolyl-endopeptidase, enzyme-clé du catabolisme de ces peptides, augmente considérablement (+ 100 %) dans le cerveau des malades d'Alzheimer (Experientia, 46, 94, 1990). C'est pourquoi, il a été suggéré et démontré que des inhibiteurs de la prolyl-endopeptidase peuvent s'opposer aux déficits de la mémoire en favorisant l'effet promnésique de certains neuropeptides et notamment de la vasopressine. Ces résultats ont été observés notamment lors d'amnésie expérimentale à la scopolamine. Pour les composés inhibiteurs étudiés, une excellente corrélation entre l'effet inhibiteur de la prolyl-endopeptidase et l'effet facilitateur de l'apprentissage a été rapportée (EP 321 956).

Il était donc particulièrement intéressant de synthétiser de nouveaux composés possédant une activité inhibitrice de la prolylendopeptidase (ou post-prolyl cleaving enzyme : PPCE).

Les composés de la présente invention, outre le fait qu'ils soient nouveaux, se sont montrés particulièrement intéressants par l'intensité et la durée de leurs propriétés à inhiber la prolyl-endopeptidase donc à éviter la dégradation des neuropeptides naturels impliqués dans les fonctions mnésiques. Leur activité est supérieure tant in vitro qu'in vivo à celle de composés décrits dans l'art antérieur comme par exemple les composés décrits dans les brevets EP 320 753 EP 321 956 et EP 345 428 en tant qu'inhibiteurs de PPCE. Elle est également nettement supérieure à celle de nootropes de référence connus également pour inhiber l'activité de la PPCE comme par exemple les composés décrits dans le brevet EP 288 685.

Les composés de l'invention sont donc utiles pour la prévention et le traitement d'une part des troubles comportementaux, notamment de la mémoire, associés au vieillissement et aux maladies dégénératives neuronales aigües ou chroniques et d'autre part des troubles psychiques associés à l'anxiété et la dépression.

L'invention concerne plus particulièrement les composés de formule générale (I) : 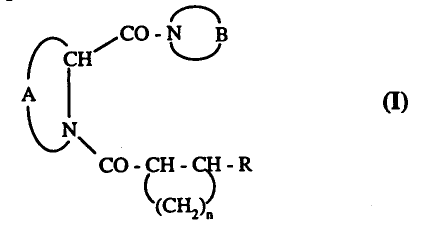 dans laquelle :
- A: représente avec les atomes de carbone et d'azote auquel il est attaché l'un quelconque des hétérocycles suivants : 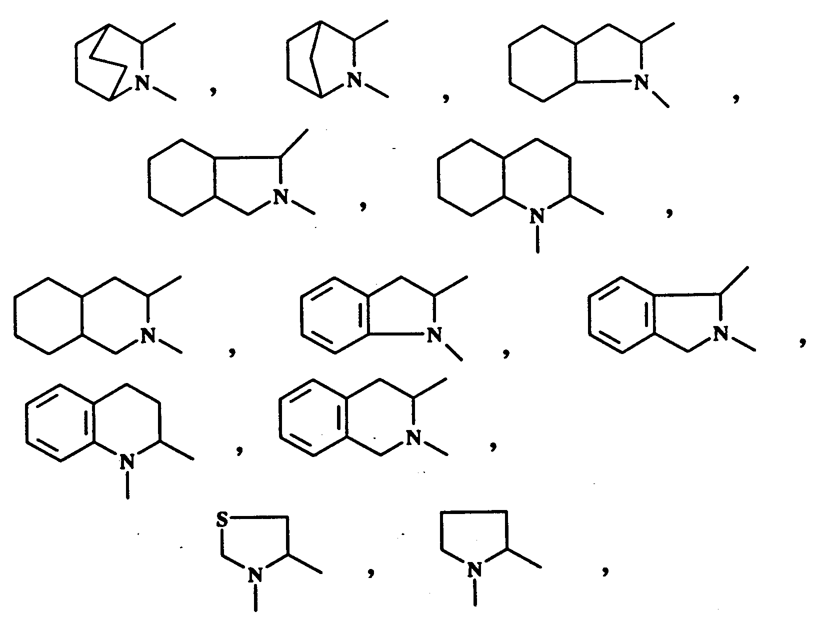
- B: représente avec l'atome d'azote auquel il est attaché l'un quelconque des hétérocycles suivants : 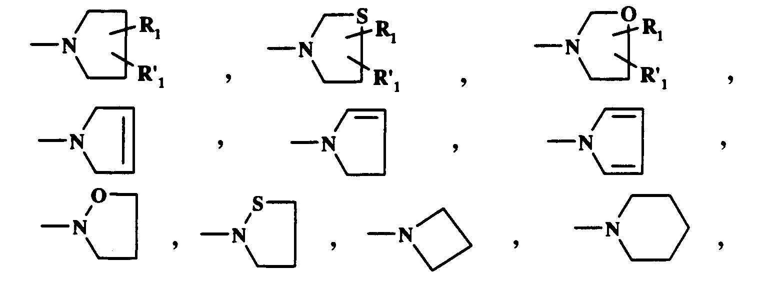 dans lesquels R₁ ou R′₁, identiques ou différents, représentent un atome d'hydrogène, un groupement alkyle (C₁-C₆) linéaire ou ramifié, hydroxy ou forment ensemble un groupement oxo,
- n: est égal à 1, 2, 3 ou 4,
- R: représente :
- un groupement phényle substitué par au moins un atome d'halogène ou un groupement alkyle (C₁-C₆) linéaire ou ramifié, hydroxy, alkoxy (C₁-C₆) linéaire ou ramifié, trifluorométhyle, nitro, amino, cyano, carboxy, alkoxycarbonyl (C₁-C₆) linéaire ou ramifié,
- un groupement benzyle,
- un groupement thiényle,
- ou, un groupement pyridyle,
leurs énantiomères, diastéréoisomères et épimères ainsi que leurs sels d'addition à un acide pharmaceutiquement acceptable.

Parmi les acides pharmaceutiquement acceptables, on peut citer à titre non limitatif les acides chlorhydrique, sulfurique, tartrique, maléique, fumarique, méthane sulfonique, camphorique, etc...

L'invention s'étend également au procédé de préparation des composés de formule (I) caractérisé en ce que l'on fait réagir un acide de formule (II), dont on a éventuellement séparé les isomères par une technique classique de séparation : 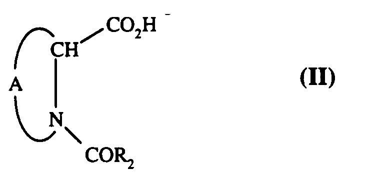 dans laquelle A a la même signification que dans la formule (I) et R₂ représente un groupement alkoxy (C₁-C₆) linéaire ou ramifié ou benzyloxy,
sur une amine de formule (III) (dont on a éventuellement séparé les isomères par une technique classique de séparation) selon une technique de couplage peptidique comme celle décrite par W. KONIG et R. GEIGER (Ber, 103, 788, 1970): 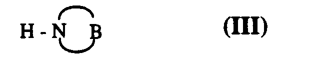 dans laquelle B a la même signification que dans la formule (I),
pour conduire au composé de formule (IV): 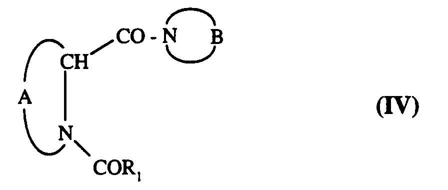 dans laquelle A, B et R₁ ont la même signification que précédemment,
que l'on déprotège par une technique classique de déprotection, pour conduire à l'amine de formule (V): 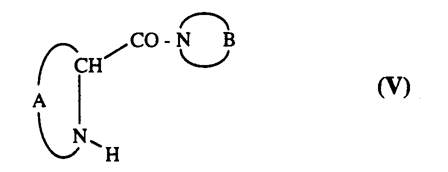 dans laquelle A et B ont la même signification que dans la formule (I),
que l'on fait réagir avec un acide de formule (VI), dont on a éventuellement séparé les isomères selon une technique classique de séparation, en présence d'un agent de couplage classique de la synthèse peptidique : 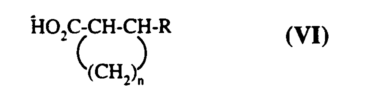 dans laquelle R et n ont la même signification que dans la formule (I),
pour conduire au composé de formule (I) que l'on purifie, le cas échéant, par une technique classique de purification, dont on sépare, si on le souhaite, les isomères par une technique classique de séparation et que l'on transforme, si nécessaire, en ses sels d'addition à un acide pharmaceutiquement acceptable.

Les composés de formule (I) peuvent également être obtenus selon le procédé caractérisé en ce que l'on fait réagir un acide de formule (VII), dont on a éventuellement séparé les isomères par une technique classique de séparation : 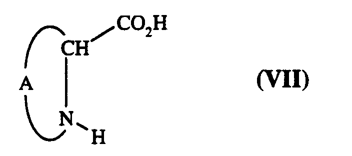 dans laquelle A a la même signification que dans la formule (I),
sur un chlorure d'acide de formule (VIII), dont on a éventuellement séparé les isomères selon une technique classique de séparation : 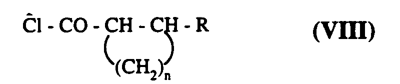 dans laquelle R et n ont la même signification que dans la formule (I),
pour conduire aux composé de formule (IX) : 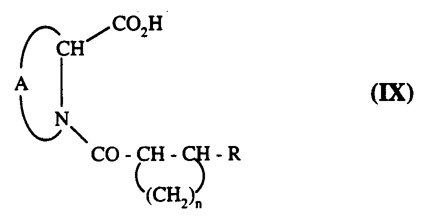 dans laquelle A, R et n ont la même signification que dans la formule (I),
que l'on fait réagir sur une amine de formule (III) (dont on a éventuellement séparé les isomères par une technique classique de séparation) selon une technique de couplage peptidique comme celle décrite par W. KONIG et R. GEIGER (Ber., 103, 788, 1970): 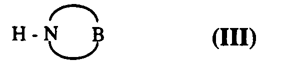 dans laquelle B a la même signification que dans la formule (I),
pour conduire au composé de formule (I), que l'on purifie, le cas échéant, par une technique classique de purification, dont on sépare, si on le souhaite, les isomères par une technique classique de séparation et que l'on transforme, si nécessaire, en ses sels d'addition à un acide pharmaceutiquement acceptable.

La séparation des isomères des composés de formule (VI) ou (VIII) peut être réalisée selon la technique décrite dans "Optical resolution for Chemical compounds" (vol. 2, part I/Optical Resolution information center, Manhattan College, Riverdale - N.Y. 10471, USA).

Les composés de formule (VI) ou (VIII), pour lesquels n = 1, sont obtenus sous forme d'énantiomères purs, par synthèse stéréoselective, selon la technique décrite par J. Vallgårda et Coll. (Tet. Lett., 32 (40), 5625-5628, 1991).
La configuration préférée des composés de formule (VI) ou (VIII) est (R,R).

Les composés de l'invention possèdent des propriétés pharmacologiques très intéressantes. Ils inhibent fortement l'activité de la prolyl-endopeptidase ce qui les rend utiles pour le traitement des désordres mnésiques et cognitifs et des troubles neurocomportementaux associés au vieillissement et aux maladies dégénératives du système nerveux, aigües ou chroniques comme la maladie d'Alzheimer, de Pick, de Korsakoff, l'accident vasculaire cérébral, le traumatisme spinal ou la sclérose amyotrophique latérale. Ils sont de la même manière utiles dans le traitement des désordres psychiques associés à l'anxiété et à la dépression.

La présente invention a également pour objet les compositions pharmaceutiques renfermant comme principe actif au moins un composé de formule générale (I) ou un de ses sels d'addition à un acide pharmacologiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie varie selon l'âge et le poids du patient, la nature et la sévérité de l'affection ainsi que la voie d'administration.
Celle-ci peut être orale, nasale, rectale ou parentérale. D'une manière générale, la posologie unitaire s'échelonne entre 0,5 et 100 mg pour un traitement en 1 à 3 prises par 24 heures.

Les exemples suivants illustrent l'invention et ne la limitent en aucune façon.

Les préparations A à F ne conduisent pas aux composés de l'invention mais à des produits de départ utiles dans la préparation des dérivés de formule (I).

Les composés des préparations A à E ont été synthétisés selon la technique décrite par J. VALLGÅRDA et coll. (Tet. Lett., 32 (40), 5625-5628, 1991).

### Préparation A : Acide (1R,2R)-2-(4-fluorophényl)cyclopropanecarboxylique

Pouvoir rotatoire : [α]_{D}²¹ = -292,5° (C = 1 %, CH₂Cl₂)

| Microanalyse élémentaire : | | |
|---|---|---|
| | C % | H % |
| calculé | 66,66 | 5,03 |
| trouvé | 66,57 | 5,02 |

### Préparation B : Acide (1R,2R)-2-(2-thiényl)cyclopropanecarboxylique

### Préparation C : Acide (1R,2R)-2-(3-trifluorométhylphényl)cyclopropane carboxylique

| Microanalyse élémentaire : | | |
|---|---|---|
| | C % | H % |
| calculé | 57,40 | 3,94 |
| trouvé | 57,21 | 4,49 |

### Préparation D : Acide (1R,2R)-2-(4-méthylphényl)cyclopropanecarboxylique

### Préparation E : Acide (1R,2R)-2-(4-méthoxyphényl)cyclopropanecarboxylique

### Préparation F : Acide (cis)-(2-benzyl)cyclopropanecarboxylique et acide (trans)-(2-benzyl)cyclopropanecarboxylique

Les isomères cis et trans de l'acide (2-benzyl)cyclopropanecarboxylique ont été préparés par séparation chromatographique sur colonne de silice en utilisant comme éluant un mélange toluène/cyclohexane (90/10), du (2-benzyl)cyclopropanecarboxylate d'éthyle. Cette séparation est suivie d'une saponification par de la soude en milieu éthanolique.

### Exemple 1 : (2S,3aS,7aS)-1-{(1R,2R)-[2-(4-Fluorophényl)cycloprop-1-yl] carbonyl}-2-[(pyrrolidin-1-yl)carbonyl]perhydroindole

### Stade A : (2S,3aS,7aS)-1-tert-Butyloxycarbonyl-2-[(pyrrolidin-1-yl) carbonyl]perhydroindole

En utilisant la technique de couplage peptidique (dicyclohexyl-carbodiimide/hydroxybenzotriazole-DCC/HOBT) décrite par W. KONIG et R. GEIGER (Ber., 103, 788, 1970) et le diméthylformamide comme solvant, on prépare à partir de pyrrolidine et d'acide (2S,3aS,7aS)-1-tertbutyloxycarbonylperhydroindole-2-carboxylique, le produit attendu qui est purifié par cristallisation dans l'acétate d'éthyle.
Point de fusion : 149 °C

### Stade B : (2S,3aS,7aS)-2-[(Pyrrolidin-1-yl)carbonyl]perhydroindole

30 mmoles du composé décrit au stade A sont dissoutes dans 100 ml d'acétate d'éthyle. En maintenant la température à 20°C, on fait passer un courant d'acide chlorhydrique et l'agitation est maintenue 18 heures à température ambiante. Le solvant est évaporé et le résidu repris par 100 ml d'eau. L'insoluble est filtré et le filtrat, après alcalinisation par addition de bicarbonate de sodium, est amené à sec. Le résidu est enfin repris successivement par 100 ml d'éthanol, 100 ml de dichlorométhane puis 100 ml d'éther éthylique. Le produit attendu est obtenu après filtration des sels et évaporation.
Point de fusion : 83 °C

### Stade C : (2S,3aS,7aS)-1-{(1R,2R)-[2-(4-Fluorophényl)cycloprop-1-yl] carbonyl}-2-[(pyrrolidin-1-yl)carbonyl]perhydroindole

Le produit attendu est obtenu selon le même procédé que celui décrit au stade A en utilisant le composé décrit au stade B et l'acide décrit dans la préparation A.
Il est purifié par chromatographie sur colonne de silice en utilisant comme éluant un mélange dichlorométhane/méthanol (97/3).
Point de fusion : 154°C Pouvoir rotatoire : [α]_{D}²² = -127,5° (C = 1 %, CH₂Cl₂)

| Microanalyse élémentaire : | | | |
|---|---|---|---|
| | C % | H % | N % |
| calculé | 71,85 | 7,60 | 7,29 |
| trouvé | 71,58 | 7,82 | 7,47 |

Les exemples 2 à 8 ont été synthétisés selon le procédé décrit dans l'exemple 1 en utilisant les produits de départ correspondants.

### Exemple 2 : (3S)-2-{(1R,2R)-[2-(4-Fluorophényl)cycloprop-1-yl]carbonyl}-3-[(pyrrolidin-1-yl)carbonyl]-2-azabicyclo[2.2.2]octane

Point de fusion : 195°C
Pouvoir rotatoire : [α]_{D}²² = -132,5° (C = 1 %, CH₂Cl₂)

| Microanalyse élémentaire : | | | |
|---|---|---|---|
| | C % | H % | N % |
| calculé | 71,33 | 7,35 | 7,56 |
| trouvé | 70,45 | 7,31 | 7,75 |

### Exemple 3 : (2S,3aS,7aS)-1-{(1R,2R)-[2-(2-Thiényl)cycloprop-1-yl] carbonyl}-2-[(pyrrolidin-1-yl)carbonyl]perhydroindole

Point de fusion : 157 °C
Pouvoir rotatoire : [α]_{D}²³ = -125,3° (C=1 %, CH₂Cl₂)

| Microanalyse élémentaire : | | | | |
|---|---|---|---|---|
| | C % | H % | N % | S% |
| calculé | 67,71 | 7,58 | 7,52, | 8,61 |
| trouvé | 67,26 | 7,61 | 7,72 | 8,47 |

### Exemple 4 : (3S)-2-{(1R,2R)-[2-(2-Thiényl)cycloprop-1-yl]carbonyl}-3-[(pyrrolidin-1-yl)carbonyl]-2-azabicyclo[2.2.2]octane

Point de fusion : 158°C
Pouvoir rotatoire : [α]_{D}²² = -130,5° (C = 1 %, CH₂Cl₂)

| Microanalyse élémentaire : | | | | |
|---|---|---|---|---|
| | C % | H % | N % | S % |
| calculé | 67,01 | 7,31 | 7,81 | 8,94 |
| trouvé | 66,34 | 7,39 | 7,85 | 8,70 |

### Exemple 5 : (2S,3aS,7aS)-1-{(1R,2R)-[2-(3-Trifluorométhylphényl)cycloprop-1-yl]carbonyl}-2-[(pyrrolidin-1-yl)carbonyl]perhydroindole

Point de fusion : 208°C
Pouvoir rotatoire : [α]_{D}²² = -130,6° (C = 1 %, CH₂Cl₂)

| Microanalyse élémentaire : | | | |
|---|---|---|---|
| | C % | H % | N % |
| calculé | 66,34 | 6,73 | 6,45 |
| trouvé | 66,37 | 6,85 | 6,83 |

### Exemple 6 : (3S)-2-{(1R,2R)-[2-(3-Trifluorométhylphényl)cycloprop-1-yl] carbonyl}-3-[(pyrrolidin-1-yl)carbonyl]-2-azabicyclo[2.2.2]octane

Point de fusion : 154°C
Pouvoir rotatoire : [α]_{D}²² = -129,5° (C = 1 %, CH₂Cl₂)

| Microanalyse élémentaire : | | | |
|---|---|---|---|
| | C % | H % | N % |
| calculé | 65,70 | 6,47 | 6,66 |
| trouvé | 65,52 | 6,25 | 6,74 |

### Exemple 7 : (2S,3aS,7aS)-1-{(1R,2R)-[2-(4-Méthylphényl)cycloprop-1-yl] carbonyl}-2-[(pyrrolidin-1-yl)carbonyl]perhydroindole

Point de fusion : 142°C

| Microanalyse élémentaire : | | | |
|---|---|---|---|
| | C % | H % | N % |
| calculé | 75,75 | 8,48 | 7,36 |
| trouvé | 74,42 | 8,62 | 7,36 |

### Exemple 8 : (3S)-2-{(1R,2R)-[2-(4-Méthoxyphényl)cycloprop-1-yl]carbonyl}-3-[(pyrrolidin-1-yl)carbonyl]-2-azabicyclo[2.2.2]octane

Point de fusion : 212°C

| Microanalyse élémentaire : | | | |
|---|---|---|---|
| | C % | H % | N % |
| calculé | 72,22 | 7,91 | 7,32 |
| trouvé | 71,60 | 7,90 | 7,21 |

### Exemple 9 : (3S)-2-[trans-(2-Benzylcycloprop-1-yl)carbonyl]-3-[(pyrrolidin-1-yl)carbonyl]-2-azabicyclo[2.2.2]octane, isomère α

et

### Exemple 10 : (3S)-2-[trans-(2-Benzylcycloprop-1-yl)carbonyl]-3-[(pyrrolidin-1-yl)carbonyl]-2-azabicyclo[2.2.2]octane, isomère β

Les isomères α et β ont été séparés par chromatographie sur colonne de silice en utilisant comme éluant un mélange dichlorométhane/acétate d'éthyle (50/50).

### Exemple 9 :

Pouvoir rotatoire : [α]_{D}²² = +27,9° (C = 1 %, CH₂Cl₂)

| Microanalyse élémentaire : | | | |
|---|---|---|---|
| | C % | H % | N % |
| calculé | 75,38 | 8,25 | 7,64 |
| trouvé | 75,45 | 8,61 | 7,31 |

### Exemple 10 :

Pouvoir rotatoire : [α]_{D}²² = -28,2° (C = 1 %, CH₂Cl₂)

| Microanalyse élémentaire : | | | |
|---|---|---|---|
| | C % | H % | N % |
| calculé | 75,38 | 8,25 | 7,64 |
| trouvé | 75,41 | 8,44 | 7,52 |

### Exemple 11 : (2S,3aS,7aS)-1-[cis-(2-Benzylcycloprop-1-yl)carbonyl]-2-[(pyrrolidin-1-yl)carbonyl]perhydroindole, isomère α

et

### Exemple 12 : (2S,3aS,7aS)-1-[cis-(2-Benzylcycloprop-1-yl)carbonyl]-2-[(pyrrolidin-1-yl)carbonyl]perhydroindole, isomère β

Les isomères α et β ont été séparés par chromatographie sur colonne de silice en utilisant comme éluant un mélange dichlorométhane/acétate d'éthyle (95/5).

### Exemple 11 :

Point de fusion : 145°C
Pouvoir rotatoire : [α]_{D}²² = -47,3° (C = 1 %, CH₂Cl₂)

| Microanalyse élémentaire : | | | |
|---|---|---|---|
| | C % | H % | N % |
| calculé | 75,75 | 8,48 | 7,36 |
| trouvé | 75,95 | 8,24 | 6,98 |

### Exemple 12 :

### Huile

Pouvoir rotatoire : [α]_{D}²² = -21,6° (C = 1 %, CH₂Cl₂)

| Microanalyse élémentaire : | | | |
|---|---|---|---|
| | C % | H % | N % |
| calculé | 75,75 | 8,48 | 7,36 |
| trouvé | 75,14 | 8,18 | 7,20 |

### Exemple 13 : (2S,3aS,7aS)-1-[trans(2-Benzylcycloprop-1-yl)carbonyl]-2-[(pyrrolidin-1-yl)carbonyl]perhydroindole, isomère α

et

### Exemple 14 : (2S,3aS,7aS)-1-[trans(2-Benzylcycloprop-1-yl)carbonyl]-2-[(pyrrolidin-1-yl)carbonyl]perhydroindole, isomère β

Les isomères α et β ont été séparés par chromatographie sur colonne de silice en utilisant comme éluant un mélange dichlorométhane/acétate d'éthyle (50/50).

### Exemple 13 :

### Huile

Pouvoir rotatoire : [α]_{D}²² = +37,5° (C = 1 %, CH₂Cl₂)

| Microanalyse élémentaire : | | | |
|---|---|---|---|
| | C % | H % | N % |
| calculé | 75,75 | 8,48 | 7,36 |
| trouvé | 75,37 | 8,94 | 6,93 |

### Exemple 14 :

### Huile

Pouvoir rotatoire : [α]_{D}²² = -14,2° (C = 1 %, CH₂Cl₂)

| Microanalyse élémentaire : | | | |
|---|---|---|---|
| | C % | H % | N % |
| calculé | 75,75 | 8,48 | 7,36 |
| trouvé | 75,28 | 8,46 | 7,34 |

### Etude pharmacologique des dérivés de l'invention

### Exemple 15 : Mesure de l'activité anti-prolyl-endopeptidase

La méthode de YOSHIMOTO et TSURU (Agr. Biol. Chem., 42, 2417, 1978) a été utilisée. La prolyl-endopeptidase est isolée de Flavobacterium meningosepticum. Le substrat enzymatique est la Z-Gly-Pro-pNitroaniline.

Un mélange de tampon phosphate 0,1 M (pH 7 ; 0,99 ml), de substrat 2 M (0,25 ml) et d'une solution du composé inhibiteur à tester (0,01 ml) est incubé à 37°C durant 5 minutes. Puis 0,1 ml d'une solution de prolyl-endopeptidase (0,5 U/ml) est ajouté et le mélange est laissé 10 minutes à 37°C. La réaction enzymatique est stoppée par addition de 2 ml d'une solution de Triton X-100-Tampon acétate 1 M (pH 4). Après repos à température ambiante, l'absorbance est mesurée à 410 nm.

Une mesure témoin est réalisée en remplaçant la solution du composé à tester par le même volume de tampon phosphate. Le pourcentage d'inhibition de l'enzyme est calculé à partir de cette mesure témoin. Pour chaque composé testé, la concentration inhibant de 50 % la prolyl-endopeptidase (IC₅₀) est déterminée.

Les composés de l'invention ont été comparés aux deux composés de référence connus pour leur forte activité inhibitrice de la prolyl-endopeptidase et décrits initialement dans ce but : (S)-1-[N-4-chlorobenzylsuccinamoyl]pyrrolidine-2-carbaldéhyde (EP 345 428) appelé ci-dessous ONO et N-(4-phénylbutanoyl)-L-thiaprolylpyrrolidineimide (exemple 3 : EP 321 956) appelé ci-dessous ZERIA).

Les dérivés de l'invention exercent une forte activité inhibitrice de la prolyl-endopeptidase, supérieure aux composés de référence comme l'indique le tableau des IC₅₀ :

| | |
|---|---|
| ZERIA | 5,4.10⁻⁷ M |
| ONO | 5,7.10⁻⁷ M |
| Exemple 1 | 1,4.10⁻⁸ M |
| Exemple 2 | 4.10⁻⁸ M |
| Exemple 3 | 1,4.10⁻⁸ M |
| Exemple 6 | 3,0.10⁻⁹ M |

### Exemple 16 : Inhibition de la prolyl-endopeptidase

La mesure de l'inhibition de la prolyl-endopeptidase a été réalisée in vitro et in vivo dans le cortex cérébral du Rat, grâce à un substrat proche d'un peptide naturellement clivé par la prolyl-endopeptidase, la Thyrotropin-Releasing Hormone (TRH).

Un extrait de cortex cérébral est préparé en broyant le tissu dans un tampon phosphate (NaH₂PO₄ 25 mM ; DTT 2 mM ; EDTA-2K 0,5 mM) à raison de 5 ml/g.

Après centrifugation, 200 µl de surnageant sont incubés 15 minutes à 37°C dans un volume final de 2,1 ml auquel sont ajoutés 250 µl de substrat (TRH-pNitroaniline) pour une nouvelle incubation de 20 minutes suivie d'une lecture immédiate d'absorbance à 410 nm.

Le produit testé est soit ajouté à l'extrait de cortex avant la première incubation (détermination de l'IC₅₀ in vitro) soit administré par voie IP 30 minutes avant la préparation de l'extrait tissulaire.

Dans les conditions du test, les composés de l'invention inhibent fortement la prolyl-endopeptidase du cortex cérébral de Rat tant in vitro qu'après administration in vivo. Cette activité est là encore très supérieure à celle des composés de référence.

| | IC₅₀ in vitro | Inhibition in vivo | |
|---|---|---|---|
| | | dose (mg/kg) | % |
| ZERIA | 5,4.10⁻⁸ M | 30 | 47 |
| | | 10 | 16 |
| ONO | 5,7.10⁻⁸ M | 30 | 19 |
| | | 10 | 5 |
| Exemple 1 | 1,1.10⁻⁹ M | 1 | 54 |
| Exemple 2 | 4.10⁻⁹ M | 1 | 42 |
| Exemple 3 | 1,4.10⁻⁹ M | 1 | 59 |
| Exemple 4 | 4,8.10⁻⁹ M | 3 | 23 |
| Exemple 5 | 1.10⁻⁹ M | 1 | 58 |
| Exemple 6 | 3,2.10⁻⁹ M | 1 | 49 |
| Exemple 14 | 2,5.10⁻⁹ M | 1 | 41 |

### Exemple 17 : Durée d'action

La durée d'action des composés vis-à-vis de l'inhibition in vivo de la prolyl-endopeptidase dans le cortex cérébral du Rat a été estimée selon la même méthodologie que dans l'exemple 16, en administrant par voie IP les composés étudiés à divers temps avant la préparation de l'extrait tissulaire. Le temps pour lequel l'effet inhibiteur est égal à 50 % de l'effet maximum (mesuré à 30 minutes) a été calculé (t_{1/2}).

De même, la biodisponibilité orale des composés de l'invention a été estimée en les administrant par voie IP ou orale 30 minutes ou 60 minutes avant la mesure de l'effet inhibiteur. L'index de biodisponibilité orale (IBO) a été déterminé en divisant l'effet inhibiteur (%) mesuré après administration orale par celui mesuré après administration IP. Les composés les plus actifs de l'invention exercent leurs effet selon une durée d'action cérébrale bien plus longue que celle des composés de référence et avec une biodisponibilité orale bien plus importante, ce qui leur confère un intérêt thérapeutique prépondérant.

| | dose (mg/kg) | t_{1/2} | IBO |
|---|---|---|---|
| ZERIA | 30 | 1 heure | 0,30 |
| ONO | 30 | 1 heure | <0,30 |
| Exemple 1 | 1 | 7 heures | 0,90 |
| Exemple 2 | 1 | 7 heures | 0,78 |
| Exemple 6 | 1 | 3 heures | 0,43 |

### Composition pharmaceutique

### Exemple 18 : Comprimé : Formule de préparation pour 1000 comprimés dosés à 10 mg

| | |
|---|---|
| Composé de l'exemple 1 | 10 g |
| Hydroxypropyl cellulose | 2 g |
| Amidon de blé | 10 g |
| Lactose | 100 g |
| Stéarate de magnésium | 3 g |
| Talc | 3 g |

## Revendications

1. Composés de formule (I) : 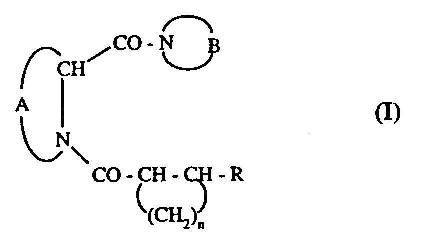 dans laquelle :
A représente avec les atomes de carbone et d'azote auquel il est attaché l'un quelconque des hétérocycles suivants : 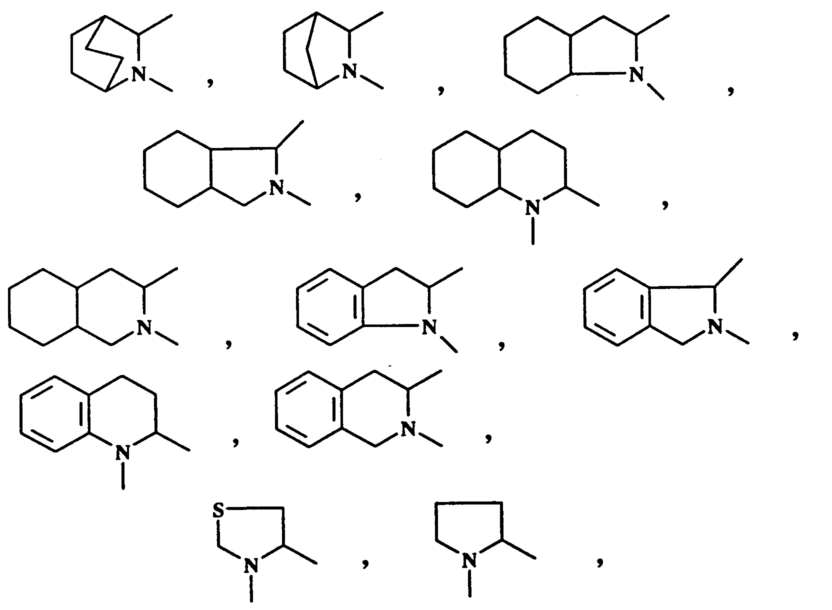
B représente avec l'atome d'azote auquel il est attaché l'un quelconque des hétérocycles suivants : 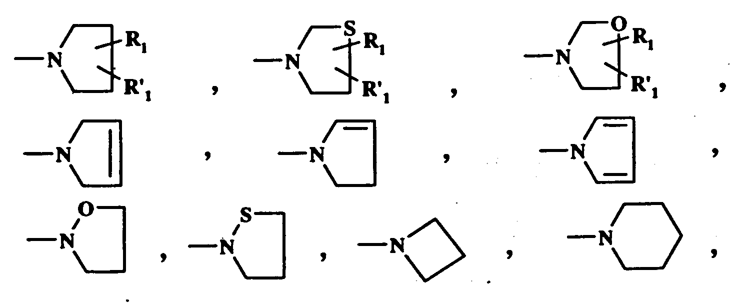 dans lesquels R₁ ou R′₁, identiques ou différents, représentent un atome d'hydrogène, un groupement alkyle (C₁-C₆) linéaire ou ramifié, hydroxy ou forment ensemble un groupement oxo,
n est égal à 1, 2, 3 ou 4,
R représente :
- un groupement phényle substitué par au moins un atome d'halogène ou un groupement alkyle (C₁-C₆) linéaire ou ramifié, hydroxy, alkoxy (C₁-C₆) linéaire ou ramifié, trifluorométhyle, nitro, amino, cyano, carboxy, alkoxycarbonyl (C₁-C₆) linéaire ou ramifié,
- un groupement benzyle,
- un groupement thiényle,
- ou, un groupement pyridyle,
leurs énantiomères, diastéréoisomères et épimères ainsi que leurs sels d'addition à un acide pharmaceutiquement acceptable.

2. Composés de formule (I) selon la revendication 1 tels que A représente avec les atomes de carbone et d'azote auxquels il est attaché un cycle 2-azabicyclo[2.2.2]octane, leurs énantiomères, diastéréoisomères et épimères ainsi que leurs sels d'addition à un acide pharmaceutiquement acceptable.

3. Composés de formule (I) selon la revendication 1 tels que A représente avec les atomes de carbone et d'azote auquel il est attaché un cycle perhydroindole, leurs énantiomères, diastéréoisomères et épimères ainsi que leurs sels d'addition à un acide pharmaceutiquement acceptable.

4. Composés de formule (I) selon la revendication 1 tels que B représente avec l'atome d'azote auquel il est attaché un cycle pyrrolidine, leurs énantiomères, diastéréoisomères et épimères ainsi que leurs sels d'addition à un acide pharmaceutiquement acceptable.

5. Composés de formule (I) selon la revendication 1 tels que R représente un groupement phényl substitué par au moins un atome d'halogène ou un groupement alkyle (C₁-C₆) linéaire ou ramifié, hydroxy, alkoxy (C₁-C₆) linéaire ou ramifié, trifluorométhyle, nitro, amino, cyano, carboxy, alkoxycarbonyl (C₁-C₆) linéaire ou ramifié, leurs énantiomères, diastéréoisomères et épimères ainsi que leurs sels d'addition à un acide pharmaceutiquement acceptable.

6. Composés de formule (I) selon la revendication 1 tels que R représente un groupement benzyle, leurs énantiomères, diastéréoisomères et épimères ainsi que leurs sels d'addition à un acide pharmaceutiquement acceptable.

7. Composés de formule (I) selon la revendication 1 tels que R représente un groupement thiényle, leurs énantiomères, diastéréoisomères et épimères ainsi que leurs sels d'addition à un acide pharmaceutiquement acceptable.

8. Composé de formule (I) selon la revendication 1 qui est le (2S,3aS,7aS)-1-{(1R,2R)-[2-(4-fluorophényl)cycloprop-1-yl]carbonyl}-2-[(pyrrolidin-1-yl) carbonyl]perhydroindole.

9. Composé de formule (I) selon la revendication 1 qui est le (3S)-2-{(1R,2R)-[2-(4-fluorophényl)cycloprop-1-yl]carbonyl}-3-[(pyrrolidin-1-yl)carbonyl]-2-azabicyclo[2.2.2]octane.

10. Composé de formule (I) selon la revendication 1 qui est le (3S)-2-{(1R,2R)-[2-(3-Trifluorométhylphényl)cycloprop-1-yl]carbonyl}-3-[(pyrrolidin-1-yl) carbonyl]-2-azabicyclo[2.2.2]octane.

11. Procédé de préparation des composés de formule (I) selon la revendication 1 caractérisé en ce que l'on fait réagir un acide de formule (II), dont on a éventuellement séparé les isomères par une technique classique de séparation : 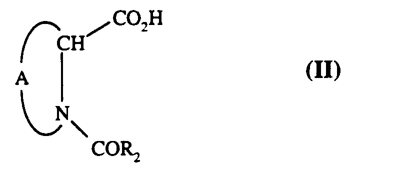 dans laquelle A a la même signification que dans la formule (I) et R₂ représente un groupement alkoxy (C₁-C₆) linéaire ou ramifié ou benzyloxy,
sur une amine de formule (III) (dont on a éventuellement séparé les isomères par une technique classique de séparation) selon une technique de couplage peptidique : 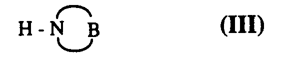 dans laquelle B a la même signification que dans la formule (I),
pour conduire au composé de formule (IV): 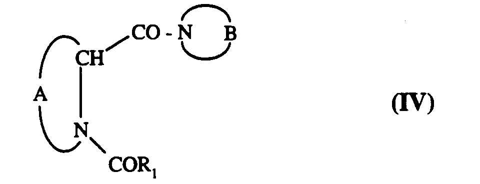 dans laquelle A, B et R₁ ont la même signification que précédemment,
que l'on déprotège par une technique classique de déprotection,
pour conduire à l'amine de formule (V) : 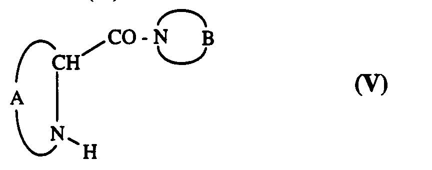 dans laquelle A et B ont la même signification que dans la formule (I),
que l'on fait réagir avec un acide de formule (VI), dont on a éventuellement séparé les isomères selon une technique classique de séparation, en présence d'un agent de couplage classique de la synthèse peptidique : 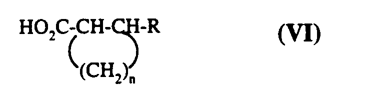 dans laquelle R et n ont la même signification que dans la formule (I),
pour conduire au composé de formule (I) que l'on purifie, le cas échéant, par une technique classique de purification, dont on sépare, si on le souhaite, les isomères par une technique classique de séparation et que l'on transforme, si nécessaire, en ses sels d'addition à un acide pharmaceutiquement acceptable.

12. Procédé de préparation des composés de formule (I) selon la revendication 1 caractérisé en ce que l'on fait réagir un acide de formule (VII), dont on a éventuellement séparé les isomères par une technique classique de séparation : 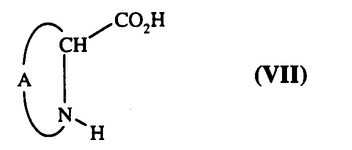 dans laquelle A a la même signification que dans la formule (I),
sur un chlorure d'acide de formule (VIII), dont on a éventuellement séparé les isomères selon une technique classique de séparation : 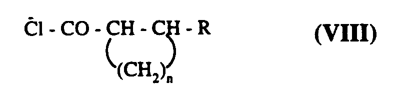 dans laquelle R et n ont la même signification que dans la formule (I),
pour conduire au composé (IX) : 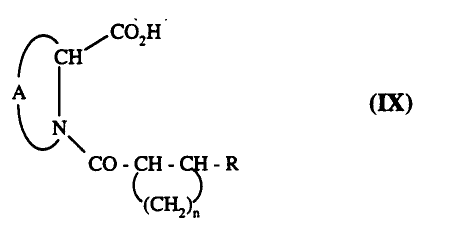 dans laquelle A, R et n ont la même signification que dans la formule (I),
que l'on fait réagir sur une amine de formule (III) (dont on a éventuellement séparé les isomères par une technique classique de séparation) selon une technique de couplage peptidique: 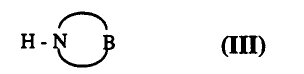 dans laquelle B a la même signification que dans la formule (I),
pour conduire au composé de formule (I), que l'on purifie, le cas échéant, par une technique classique de purification, dont on sépare, si on le souhaite, les isomères par une technique classique de séparation et que l'on transforme, si nécessaire, en ses sels d'addition à un acide pharmaceutiquement acceptable.

13. Compositions pharmaceutiques contenant comme principe actif au moins un composé selon l'une quelconque des revendications 1 à 10, seul ou en combinaison avec un ou plusieurs excipients ou véhicules inertes, non toxiques, pharmaceutiquement acceptables.

14. Compositions pharmaceutiques selon la revendication 13 contenant au moins un principe actif selon l'une des revendications 1 à 10 utiles en tant qu'inhibiteurs de prolyl-endopeptidase pour le traitement des désordres mnésiques et cognitifs et des troubles neurocomportementaux associés à la dépression, à l'anxiété, au vieillissement et aux maladies dégénératives du système nerveux, aigües ou chroniques comme la maladie d'Alzheimer, de Pick, de Korsakoff, l'accident vasculaire cérébral, le traumatisme spinal ou la sclérose amyotrophique latérale.

## Claims

1. Compounds of formula (I): 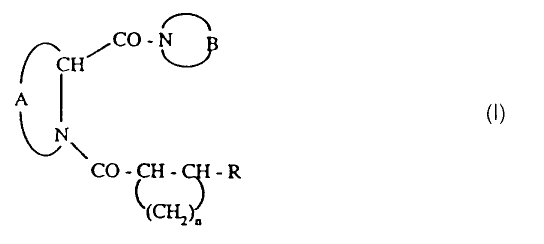 in which:
A represents, with the carbon and nitrogen atoms to which it is bonded, any one of the following heterocycles: 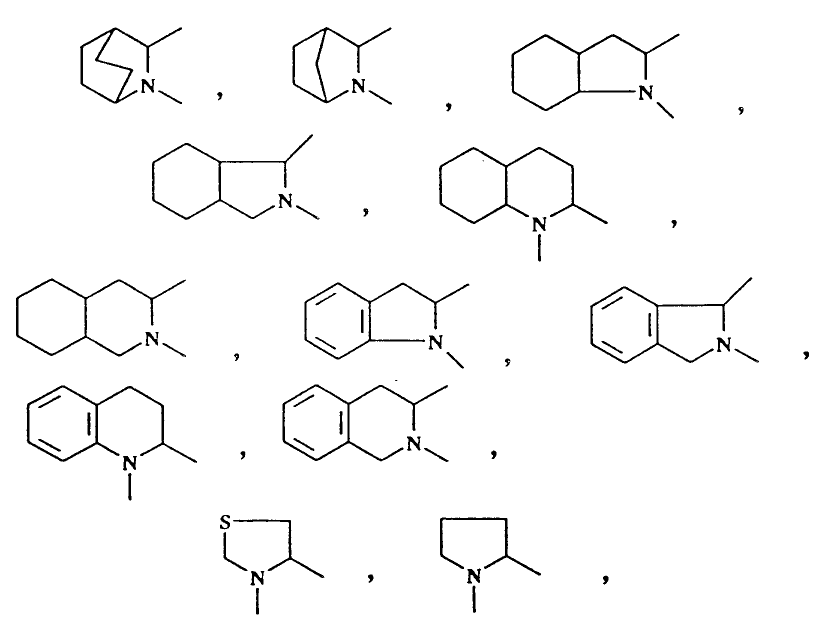
B represents, with the nitrogen atom to which it is bonded, any one of the following heterocycles: 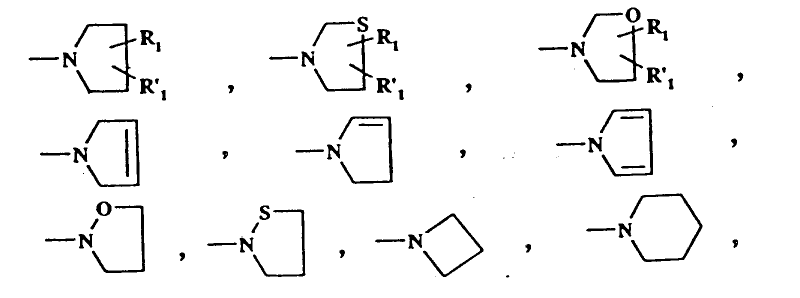 in which R₁ and R'₁, which may be the same or different, represent a hydrogen atom, a linear or branched (C₁-C₆)alkyl group or a hydroxy group, or together form an oxo group,
n is 1, 2, 3 or 4, and
R represents:
- a phenyl group substituted by at least one halogen atom or one linear or branched (C₁-C₆)alkyl, hydroxy, linear or branched (C₁-C₆)alkoxy, trifluoromethyl, nitro, amino, cyano, carboxy or linear or branched (C₁-C₆)alkoxycarbonyl group,
- a benzyl group,
- a thienyl group, or
- a pyridyl group,
their enantiomers, diastereoisomers and epimers, and addition salts thereof with a pharmaceutically acceptable acid.

2. Compounds of formula (I) according to claim 1 in which A represents, with the carbon and nitrogen atoms to which it is bonded, a 2-azabicyclo[2.2.2]octane ring, their enantiomers, diastereoisomers and epimers, and addition salts thereof with a pharmaceutically acceptable acid.

3. Compounds of formula (I) according to claim 1 in which A represents, with the carbon and nitrogen atoms to which it is bonded, a perhydroindole ring, their enantiomers, diastereoisomers and epimers, and addition salts thereof with a pharmaceutically acceptable acid.

4. Compounds of formula (I) according to claim 1 in which B represents, with the nitrogen atom to which it is bonded, a pyrrolidine ring, their enantiomers, diastereoisomers and epimers, and addition salts thereof with a pharmaceutically acceptable acid.

5. Compounds of formula (I) according to claim 1 in which R represents a phenyl group substituted by at least one halogen atom or one linear or branched (C₁-C₆)alkyl, hydroxy, linear or branched (C₁-C₆)alkoxy, trifluoromethyl, nitro, amino, cyano, carboxy or linear or branched (C₁-C₆)alkoxycarbonyl group, their enantiomers, diastereoisomers and epimers, and addition salts thereof with a pharmaceutically acceptable acid.

6. Compounds of formula (I) according to claim 1 in which R represents a benzyl group, their enantiomers, diastereoisomers and epimers, and addition salts thereof with a pharmaceutically acceptable acid.

7. Compounds of formula (I) according to claim 1 in which R represents a thienyl group, their enantiomers, diastereoisomers and epimers, and addition salts thereof with a pharmaceutically acceptable acid.

8. Compound of formula (I) according to claim 1 that is (2S,3aS,7aS)-1-{(1R,2R)-[2-(4-fluorophenyl)cycloprop-1-yl]carbonyl}-2-[(pyrrolidin-1-yl)carbonyl]perhydroindole.

9. Compound of formula (I) according to claim 1 that is (3S)-2-{(1R,2R)-[2-(4-fluorophenyl)cycloprop-1-yl]carbonyl}-3-[(pyrrolidin-1-yl)carbonyl]-2-azabicyclo[2.2.2]octane.

10. Compound of formula (I) according to claim 1 that is (3S)-2-{(1R,2R)-[2-(3-trifluoromethylphenyl)cycloprop-1-yl]carbonyl}-3-[(pyrrolidin-1-yl)carbonyl]-2-azabicyclo[2.2.2]octane.

11. Process for the preparation of compounds of formula (I) according to claim 1, characterised in that an acid of formula (II), which has optionally been separated into its isomers by a conventional separation technique: 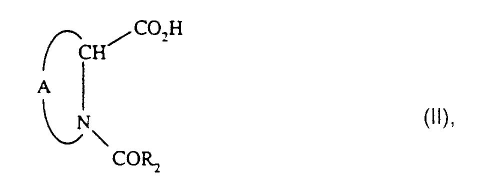 in which A is as defined in formula (I) and R₂ represents a linear or branched (C₁-C₆)alkoxy group or a benzyloxy group,
is reacted with an amine of formula (III) (which has optionally been separated into its isomers by a conventional separation technique) in accordance with a peptide coupling technique: 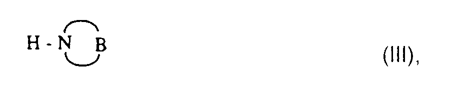 in which B is as defined in formula (I),
to yield a compound of formula (IV): 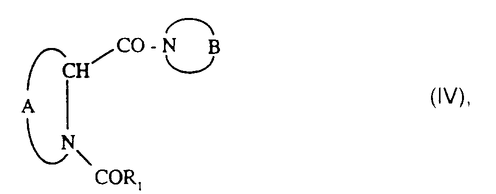 in which A, B and R₁ are as defined above,
which is deprotected by a conventional deprotecting technique to yield an amine of formula (V): 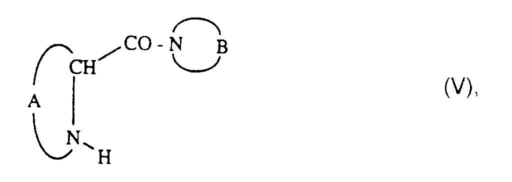 in which A and B are as defined in formula (I),
which is reacted with an acid of formula (VI), which has optionally been separated into its isomers in accordance with a conventional separation technique, in the presence of a coupling agent customary in peptide synthesis: 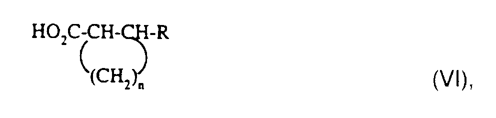 in which R and n are as defined in formula (I),
to yield a compound of formula (I), which is purified, where appropriate, by a conventional purification technique, is separated into its isomers, if desired, by a conventional separation technique, and is converted, if necessary, into its addition salts with a pharmaceutically acceptable acid.

12. Process for the preparation of compounds of formula (I) according to claim 1, characterised in that an acid of formula (VII), which has optionally been separated into its isomers by a conventional separation technique: 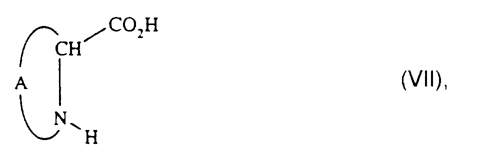 in which A is as defined in formula (I),
is reacted with an acid chloride of formula (VIII), which has optionally been separated into its isomers in accordance with a conventional separation technique: 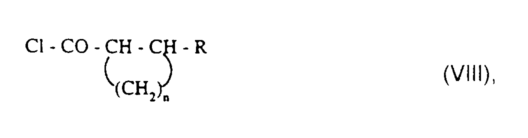 in which R and n are as defined in formula (I),
to yield a compound of formula (IX): 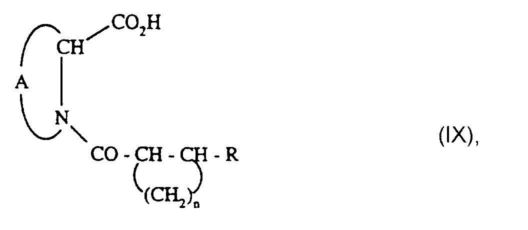 in which A, R and n are as defined in formula (I),
which is reacted with an amine of formula (III) (which has optionally been separated into its isomers by a conventional separation technique) in accordance with a peptide coupling technique: 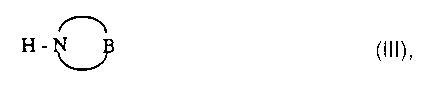 in which B is as defined in formula (I),
to yield a compound of formula (I), which is purified, where appropriate, by a conventional purification technique, is separated into its isomers, if desired, by a conventional separation technique, and is converted, if necessary, into its addition salts with a pharmaceutically acceptable acid.

13. Pharmaceutical compositions comprising as active ingredient at least one compound according to any one of claims 1 to 10, on its own or in combination with one or more inert, non-toxic, pharmaceutically acceptable excipients or carriers.

14. Pharmaceutical compositions according to claim 13 comprising at least one active ingredient according to one of claims 1 to 10, for use as prolyl-endopeptidase inhibitors in the treatment of memory and cognitive disorders and of neuro-behavioural disorders associated with depression, anxiety, ageing and acute or chronic degenerative diseases of the nervous system, such as Alzheimer's disease, Pick's disease, Korsakoff's disease, cerebrovascular accident, spinal trauma or amyotrophic lateral sclerosis.

## Patentansprüche

1. Verbindungen der Formel (I): 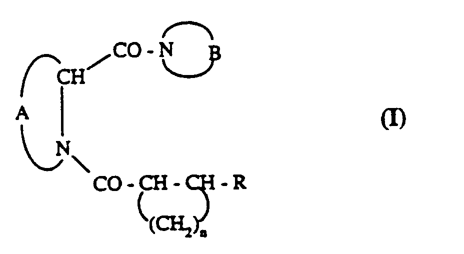 in der:
A zusammen mit den Kohlenstoff- und Stickstoffatomen, an die es gebunden ist, einen der folgenden Heterocyclen darstellt: 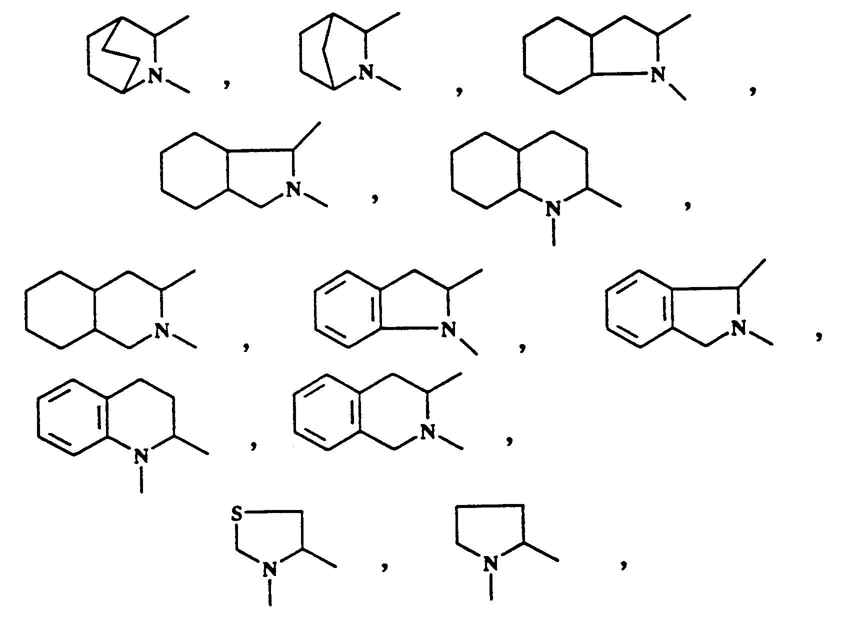
B zusammen mit dem Stickstoffatom, an das es gebunden ist, einen der folgenden Heterocyclen darstellt: 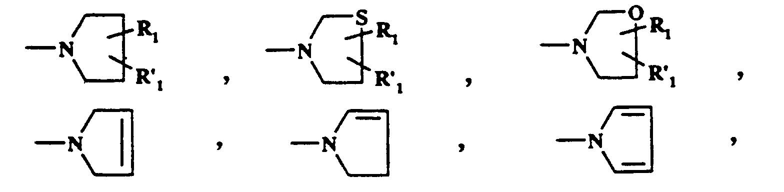 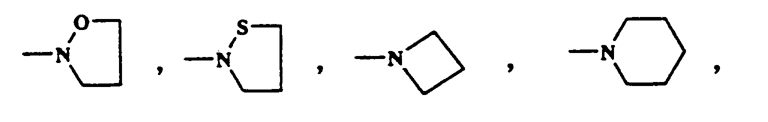 in denen R₁ oder R'₁, die gleichartig oder verschieden sein können, ein Wasserstoffatom, eine geradkettige oder verzweigte (C₁-C₆)-Alkylgruppe, eine Hydroxylgruppe oder gemeinsam eine Oxogruppe bedeuten,
n 1, 2, 3 oder 4 darstellt und
R
- eine Phenylgruppe, die durch mindestens ein Halogenatom oder eine geradkettige oder verzweigte (C₁-C₆)-Alkylgruppe, Hydroxylgruppe, geradkettige oder verzweigte (C₁-C₆)-Alkoxygruppe, Trifluormethylgruppe, Nitrogruppe, Aminogruppe, Cyanogruppe, Carboxygruppe oder geradkettige oder verzweigte (C₁-C₆)-Alkoxycarbonylgruppe substituiert ist,
- eine Benzylgruppe,
- eine Thienylgruppe oder
- eine Pyridylgruppe
darstellt,
deren Enantiomere, Diastereoisomere und Epimere sowie deren Additionssalze mit einer pharmazeutisch annehmbaren Säure.

2. Verbindungen der Formel (I) nach Anspruch 1, worin A zusammen mit den Kohlenstoff- und Stickstoffatomen, an die es gebunden ist, einen 2-Azabicyclo[2.2.2]octan-Ring darstellt, deren Enantiomere, Diastereoisomere und Epimere sowie deren Additionssalze mit einer pharmazeutisch annehmbaren Säure.

3. Verbindungen der Formel (I) nach Anspruch 1, worin A zusammen mit den Kohlenstoff- und Stickstoffatomen, an die es gebunden ist, einen Perhydroindolring darstellt, deren Enantiomere, Diastereoisomere und Epimere sowie deren Additionssalze mit einer pharmazeutisch annehmbaren Säure.

4. Verbindungen der Formel (I) nach Anspruch 1, worin B zusammen mit dem Stickstoffatom, an das es gebunden ist, einen Pyrrolidinring darstellt, deren Enantiomere, Diastereoisomere und Epimere sowie deren Additionssalze mit einer pharmazeutisch annehmbaren Säure.

5. Verbindungen der Formel (I) nach Anspruch 1, worin R eine Phenylgruppe darstellt, die durch mindestens ein Halogenatom oder eine geradkettige oder verzweigte (C₁-C₆)-Alkylgruppe, Hydroxylgruppe, geradkettige oder verzweigte (C₁-C₆)-Alkoxygruppe, Trifluormethylgruppe, Nitrogruppe, Aminogruppe, Cyanogruppe, Carboxygruppe oder geradkettige oder verzweigte (C₁-C₆)-Alkoxycarbonylgruppe substituiert ist, deren Enantiomere, Diastereoisomere und Epimere sowie deren Additionssalze mit einer pharmazeutisch annehmbaren Säure.

6. Verbindungen der Formel (I) nach Anspruch 1, worin R eine Benzylgruppe darstellt, deren Enantiomere, Diastereoisomere und Epimere sowie deren Additionssalze mit einer pharmazeutisch annehmbaren Säure.

7. Verbindungen der Formel (I) nach Anspruch 1, worin R eine Thienylgruppe darstellt, deren Enantiomere, Diastereoisomere und Epimere sowie deren Additionssalze mit einer pharmazeutisch annehmbaren Säure.

8. Verbindung der Formel (I) nach Anspruch 1, nämlich (2S,3aS,7aS)-1-{(1R,2R)-[2-(4-Fluorphenyl)-cycloprop-1-yl]-carbonyl}-2-[(pyrrolidin-1-yl)-carbonyl]-perhydroindol.

9. Verbindung der Formel (I) nach Anspruch 1, nämlich (3S)-2-{(1R,2R)-[2-(4-Fluorphenyl)-cycloprop-1-yl]-carbonyl}-3-[(pyrrolidin-1-yl)-carbonyl]-2-azabicyclo[2.2.2]octan.

10. Verbindung der Formel (I) nach Anspruch 1, nämlich (3S)-2-{(1R,2R)-[2-[3-Trifluormethylphenyl)-cycloprop-1-yl]-carbonyl}-3-[(pyrrolidin-1-yl)-carbonyl]-2-azabicyclo[2.2.2]octan.

11. Verfahren zur Herstellung der Verbindungen der Formel (I) nach Anspruch 1, dadurch gekennzeichnet, daß man eine Säure der Formel (II), die man gegebenenfalls mit Hilfe klassischer Trennungsmethoden in die Isomeren aufgetrennt hat: 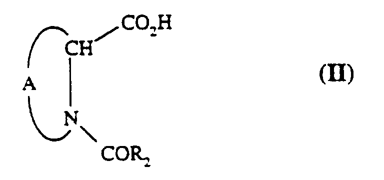 in der A die bezüglich der Formel (I) angegebenen Bedeutungen besitzen und R₂ eine geradkettige oder verzweigte (C₁-C₆)-Alkoxygruppe oder eine Benzyloxygruppe darstellt,
mit einem Amin der Formel (III) (das man gegebenenfalls mit Hilfe einer klassischen Trennmethode in die Isomeren aufgetrennt hat): 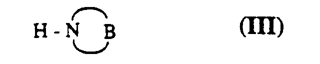 in der B die bezüglich der Formel (I) angegebenen Bedeutungen besitzt, nach einer Peptidkupplungstechnik umsetzt
zur Bildung der Verbindung der Formel (IV): 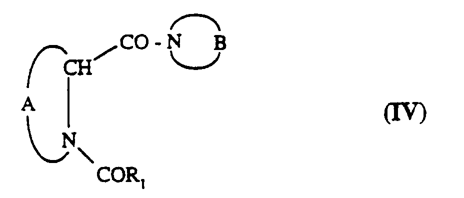 in der A, B und R₁ die oben angegebenen Bedeutungen besitzen,
von welcher man mit Hilfe einer klassischen Schutzgruppenabspaltungstechnik die Schutzgruppe abspaltet,
zur Bildung des Amins der Formel (V): 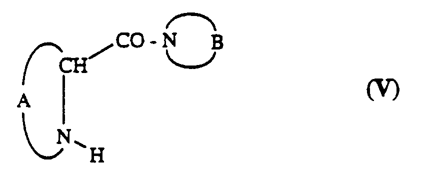 in der A und B die bezüglich der Formel (I) angegebenen Bedeutungen besitzen, welches man mit einer Säure der Formel (VI) umsetzt, die man gegebenenfalls zuvor mit Hilfe einer klassischen Trennmethode in die Isomeren aufgetrennt hat: 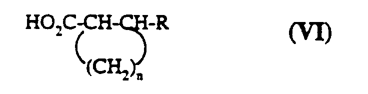 in der R und n die bezüglich der Formel (I) angegebenen Bedeutungen besitzen, in Gegenwart eines klassischen Kupplungsmittels für die Peptidsynthese umsetzt zur Bildung der Verbindung der Formel (I), welche man gegebenenfalls mit Hilfe einer klassischen Reinigungsmethode reinigt, gewünschtenfalls mit Hilfe einer klassischen Trennmethode in die Isomeren auftrennt und erforderlichenfalls mit einer pharmazeutisch annehmbaren Säure in ihre Additionssalze überführt.

12. Verfahren zur Herstellung der Verbindungen der Formel (I) nach Anspruch 1, dadurch gekennzeichnet, daß man eine Säure der Formel (VII), die man gegebenenfalls mit Hilfe einer klassischen Trennmethode in die Isomeren aufgetrennt hat: 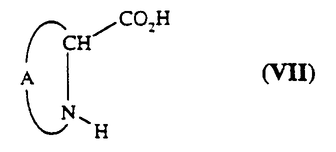 in der A die bezüglich der Formel (I) angegebenen Bedeutungen besitzt. mit einem Säurechlorid der Formel (VIII), die man gegebenenfalls mit Hilfe einer klassischen Trennmethode in die Isomeren aufgetrennt hat: 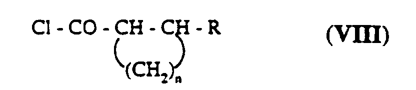 in der R und n die bezüglich der Formel (I) angegebenen Bedeutungen besitzen, umsetzt zur Bildung der Verbindung der Formel (IX): 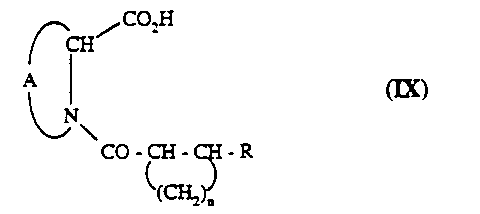 in der A, R und n die bezüglich der Formel (I) angegebenen Bedeutungen besitzen, welche man mit einem Amin der Formel (III) (welches man gegebenenfalls mit Hilfe einer klassischen Trennmethode in die Isomeren aufgetrennt hat): 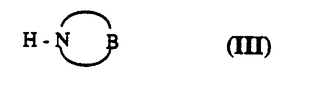 in der B die bezüglich der Formel (I) angegebenen Bedeutungen besitzt, mit Hilfe einer klassischen Peptidkupplungstechnik umsetzt zur Bildung der Verbindung der Formel (I), welche man gegebenenfalls mit Hilfe einer klassischen Reinigungsmethode reinigt, gewünschtenfalls mit Hilfe einer klassischen Trennmethode in die Isomeren auftrennt und erforderlichenfalls mit einer pharmazeutisch annehmbaren Säure in ihre Additionssalze überführt.

13. Pharmazeutische Zubereitungen enthaltend als Wirkstoff mindestens eine Verbindung nach einem der Ansprüche 1 bis 10 allein oder in Kombination mit einem oder mehreren inerten, nichttoxischen, pharmazeutisch annehmbaren Trägermaterialien oder Bindemitteln.

14. Pharmazeutische Zubereitungen nach Anspruch 13 enthaltend mindestens einen Wirkstoff nach einem der Ansprüche 1 bis 10, geeignet als Inhibitoren der Prolyl-endopeptidase bei der Behandlung von Gedächtnisstörungen und Erkenntnisstörungen und Störungen des Nervenverhaltens als Folge von Depression, Angst, Altern und akuten oder chronischen degenerativen Erkrankungen des Zentralnervensystems, wie die Alzheimersche Krankheit, die Picksche Krankheit, die Korsakoff-Krankheit, Gehirngefäßvorfällen, Rückenmarktraumata oder amyotrophische Lateralsklerose.
